Europäisches Patentamt

European Patent Office

Office européen des brevets

(19)

(11) Publication number: **0 254 862**
**A1**

## EUROPEAN PATENT APPLICATION

(12)

(21) Application number: 87108867.0

(22) Date of filing: 20.06.87

(51) Int. Cl.⁴: **A61K 39/015** , **C12N 15/00** , **C12P 21/00**

(30) Priority: 26.06.86 US 879076

(43) Date of publication of application:
03.02.88 Bulletin 88/05

(84) Designated Contracting States:
AT BE CH DE ES FR GB GR IT LI LU NL SE

(71) Applicant: BEHRINGWERKE
Aktiengesellschaft
Postfach 1140
D-3550 Marburg 1(DE)

(72) Inventor: Shaw, Alan
19 Quai de Cheval Blanc
CH-1227 Geneva(CH)
Inventor: Humbert, Yves
5 Chemin Emile Dusonchet
CH-1256 Geneva(CH)

(74) Representative: Meyer-Dulheuer,
Karl-Hermann, Dr. et al
HOECHST Aktiengesellschaft Zentrale
Patentabteilung Postfach 80 03 20
D-6230 Frankfurt/Main 80(DE)

(54) Vaccines against protozoan parasites.

(57) A composition for use as a vaccine against or a medicament for treating protozoan parasitic infection, which infection is characterized by a parasite having a surface antigen which contains a series of repeated amino acids, known as a repitope. The composition comprises an immunologically effective amount of a peptide or polypeptide characterized by a substantial or complete deletion of the repitope. These compositions are useful in a method of immunizing against or treating protozoan parasitic infection.

*FIG. I*

```
GlyGlyGlyGlyGlyTyrGlnGluLeuValLysLysLeuGluAlaLeuGluAspAlaVal
GGGGGGGGGGGGGGGTTATCAAGAACTTGTCAAAAAACTAGAAGCTTTAGAAGATGCAC

LeuThrGlyTyrSerLeuPheGlnLysGluLysMetValLeuAsnGluGlyThrSerGly
TATTGACAGGTTATAGTTTATTTCAAAAGGAAAAAATGGTATTAAATGAAGGAACAAGTG

ThrAlaValThrThrSerThrProGlySerGlyGlySerValThrSerGlyGlySerGly
GAACAGCTGTTACAACTAGTACACCTGGTTCAGGTGGTTCAGTTACTTCAGGTGGTTCAG

GlySerValAlaSerValAlaSerGlyGlySerGlyGlySerValAlaSerGlyGlySer
GTGGTTCAGTTGCTTCAGTTGCTTCAGGTGGTTCAGGTGGCTCAGTTGCTTCAGGTGGTT

GlyAsnSerArgArgThrAsnProSerAspAsnSerSerAspSerAspAlaLysSerSer
CAGGTAATTCAAGACGTACAAATCCTTCAGATAATTCAAGTGATTCAGATGCTAAATCTT

ProArg
CCCCCC
```

# VACCINES AGAINST PROTOZOAN PARASITES

## TECHNICAL FIELD OF INVENTION

This invention relates to vaccines against protozoan parasitic infections, such vaccines being characterized by immunogens that lack the repeated epitopes of the disease-causing parasite's native surface antigens. More specifically, this invention relates to immunogens which lack the repeated epitopes of various parasitic surface antigens, methods of producing them, and methods of using them, as well as compositions containing them for immunizing mammals against and treating protozoan parasitic infections.

## BACKGROUND ART

Typical protozoa are unicellular organisms which lack a cell wall and obtain food phagotropically. Protozoa are divided into groups on the basis of their mechanisms of motility. Protozoa that move by amoeboid motion are called Sarcodina; those characterized by flagella are Flagellata or Mastigophora, and those using cilia are called Ciliophora. A fourth group, the Sporozoa, are non-mobile and are characterized by their division by multiple fission of a single cell into a number of smaller cells called spores.

Sporozoa comprise a large group of protozoa, all of which are obligate parasites. They produce structures called sporozoites, which are analogous to the resting spores of bacteria or fungi. Sporozoites are involved in the transmission of the parasite to new host. One member of this class is Babesia which is found in the red blood cells of various higher animals, and which is transmitted by ticks. By far the most important member of the class is made up of plasmodia (malaria parasites).

Malaria is a major cause of morbidity and mortality in many parts of the word [3rd Annual Report on the Special Program For Research And Training in Tripical Diseases, WHO, Geneva (1979)]. Almost one third of the world's population is exposed to the risk of infection. There are an estimated 150 million cases of malaria per year and in Africa alone the annual mortality, mainly among children, is about 1 million [J. A. Deans and S. Cohen, "Immunology Of Malaria", Ann. Rev. Microbiol., 37, pp. 25-49 (1963); A. Noguer et al., WHO Chron., 32, pp. 9-17 (1978)]. Accordingly malaria constitutes a major constraint on economic progress in tropical and subtropical areas of the world.

More than 100 plasmodial species that cause malaria in a wide range of vertebrates have been described. These species display a narrowly-defined host specificity. For example, only four species infect man: P. falciparum, P. vivax, P. malariae and P. ovale. There is also a high degree of antigenic heterogeneity within each plasmodium species [E.g., R. L. Coppel et al., "Isolate-Specific S-Antigen Of Plasmodium Falciparum Contains A Repeated Sequence of Eleven Amino Acids", Nature, 306, pp. 751-56 (1983); R.F. Anders et al., Characterization Of An S-Antigen Synthesized By Several Isolates Of Plasmodium Falciparum", Proc. Natl. Acad. Sci. USA, 80, pp. 6652-56 (1983)].

Global eradication of malaria is beset by many practical difficulties. These problems have been compounded by the increasing resistance of mosquitos to insecticides and by the appearance of strains of malarial parasites that are resistant to the more commonly used chemotherapeutic agents, including 4-amino quinolines, like chloroquine.

In addition, the preparation of malarial vaccines is beset by several difficulties. For example, preparation of antigenic compounds from parasites grown in culture is very difficult, particularly on the large scale necessary for a useful vaccine. Furthermore, the life cycle of the malarial parasite presents a variety of potential targets for the action of specific immune processes. For example, protective immunity can operate against two distinct development stages of the parasite --the sporozoites and the asexual blood stages [G. V. Brown et al., "Target Antigens Of Purified Human Immunoglobulins Which Inhibit Growth Of Plasmodium Falciparum In Vitro", Nature, 297, pp. 591-93 (1982)]. In addition, malarial parasites may be able to evade detection and attack by the immune system of the compromised host by virtue of an "immune-decoy protein" located within the immunodominant region of their immunoreactive surface antigen (see discussion below) [G. N. Gordon et al., Nature, 305, pp. 29-33 (1983)].

The sporozoites of plasmodia are introduced into the body by the bite of the mosquito. The asexual blood stages are largely responsible for the clinical symptoms of malaria. They involve maturation through a number of development stages called ring, trophozoite and schizont. Finally, at the end of the asexual cycle, mature schizonts rupture the red blood cells and merozoites are released into the circulation. The released merozoites then attach to specific receptors on other red cell membranes to initiate invasion. They also differentiate

into male and female gametocytes which may be ingested by mosquitos and sexually reproduced by them to produce sporozoites for subsequent infection.

It has been shown that antigens of the asexual blood stage, and in particular of schizonts and merozoites, play a major role in protective immunity against malaria. For example, immunoglobulins from immune donors recognize schizont-merozoite specific antigens [L. H. Perrin and R. Dayal, Immunol. Rev., 61, pp. 245-69 (1982); G. V. Brown et al., Nature, 297, pp. 591-93 (1982); J. H. L. Playfair, Bull. WHO, 57, pp. 245-46 (1979)]. Immune serum and monoclonal antibodies to schizont-merozoites have also demonstrated an inhibitory activity against parasite growth in vitro. [Brown et al. supra; Playfair, supra; L. H. Perrin et al., Nature, 289, pp. 301-02 (1981); L. Schofield et al., Infect. Immun., 38, pp. 893-97 (1982)]. Moreover, the Perrin et al. monoclonal antibodies react with plasmodium isolates from various parts of the world and inhibit their grown [L. H. Perrin et al., Clin. Exp. Immunol. (1984); B. Berkli et al., Abstract Sixth Int. Cong. of Parasitology, Calgary (1984)].

In an attempt to learn more about the malarial parasite and to produce an effective vaccine against it, various malarial antigens have been cloned and produced by recombinant DNA technology. For example, a 145,000 molecular weight antigen has been reported to have been cloned in E.coli [K. G. Odink et al., "Cloning Of Malarial Genes Coding For High Molecular Weight Antigens: Isolation Of Fragments Of Plasmodium Falciparum Genes Coding For Proteins Of 145,000 Molecular Weight", Mol. Biochem. Parasite, 10, pp. 55-66 (1984). In addition, the gene encoding the circumsporozoite protein of the human malaria parasite P. vivax has been reported [D. E. Arnot et al., "Circumsporozoite Protein of Plasmodium vivax: Gene Cloning and Characterization of the Immunodominant Epitope," Science, 230, pp. 815-18 (1985)]. As a result of this research, it has been demonstrated that malaria surface antigens have regions of repeating amino acids. For example, the deduced sequence of the circumsporozoite (CS) protein P. vivax consists of 373 amino acids with a central region of 19 tandem repeats of nine amino acids.

A group of unidentified "blood stage" antigens have also been cloned and expressed in E.coli [D. J. Kemp et al., "Expression Of Plasmodium Falciparum Blood-Stage Antigens In Escherichia Coli : Detection With Antibodies From Immune Humans", Proc. Natl. Acad. Sci. USA, 80, pp. 3787-91 (1983)-]. These antigens also appear to have repeat regions. For example, one of the Kemp et al. antigens has been reported to be isolate-specific and to contain a repeated sequence of 11 amino acids [R. L. Coppel et al., "Isolate-Specific S-Antigen Of Plasmodium Falciparum Contains A Repeated Sequence Of Eleven Amino Acids", Nature, 306, pp. 751-56 (1983)].

Repeated amino acid sequences are also said to be present in other blood-stage antigens of plasmodial parasites [R. L. Coppel et al., Nature, 302, 751 (1983); R. L. Coppel et al., Nature, 310, 789 (1984); A. F. Cowman et al., Cell, 40, 775 (1985)]. For example, the circumsporozoite (CS0 proteins of sporozoites contain an immunodominant epitope that is repeated at least twice within each molecule. A malarial sporozoite surface antigen from P. Knowlesi antigen is reported to be characterized by a repeated amino acid sequence [J. Ellis et al., "Cloning And Expression In E.coli Of The Malarial Sporozoite Surface Antigen Gene From Plasmodium Knowlesi", Nature, 302, pp. 536-38 (1983)]. Furthermore, the immunoreactive region of the CS-protein of P.Knowlesi has been said to be contained within a 12-time tandemly repeating epitope containing a repeating 12 amino acid unit, which has been designated a "repitope" [G. N. Godson et al., "Identification And Chemical Synthesis Of A Tandemly Repeated Immunogenic Region of Plasmodium Knowlesi Circumsporoite Protein". Nature, 305, pp. 29-33 (1983)]. Other protozoan parasitic antigens, such as Babesia, are also characterized by such repeated epitopes [A. F. Cowman et al., Cell, 37, 653-660 (1984)].

It has been observed that the most immunogenic portion of the plasmodial parasitic antigen is the repeated sequence portion or epitope. For example, in P. falciparum, a 30-amino acid peptide derived from the tripeptide repeats is far more immunogenic than a peptide derived from the unique portion located just upstream of the repeats. The very high immunogenic character of the repeated epitopes in plasmodial antigens has also been confirmed with other species of protozoan parasite [V. Enea et al., Proc Natl. Acad. Sci. USA, 81, pp. 7520-24 (1984)]. In view of their high immunogenicity the repeated epitopes of these antigens have been the most focused upon to date as the potential sources of vaccines against malaria. However, to date, the results have been less promising than hoped.

Although the repeats are immunodominant, it has been suggested they serve as a decoy in preventing the induction of protective antibodies against parasitic infection [G. N. Godson et al., Philos. Trans. R. Soc., B307, pp. 129-32 (1984)]. For example, while the repeated epitopes of Plasmodium falciparum vary from isolate to isolate, the unique sequence upstream of the repeats remain the same, which suggests a mechanism on the part of the parasite to avoid detection [A. F. Cowman et al., Cell, 40, pp. 775-82 (1985)].

## DISCLOSURE OF THE INVENTION

The present invention solves the problems referred to above by providing vaccines against infection by protozoan parasites which parasites are characterized by surface antigenic determinants or epitopes comprising a series of repeated amino acid sequence, or repitopes. More particularly, we provide in accordance with this invention, vaccines which lack substantially all of the repitope which characterizes the native protozoan parasitic surface antigens. These vaccines are capable of eliciting antibodies in a compromised or target host, which antibodies recognize and bind to the unique sequences on either side of the deleted repeats so as to immobilize and kill the target parasite.

By virtue of our invention, it is possible for the first time to immunize against, or treat a host compromised by infection with, parasites which may have heretofore evaded targetting by the immune system through the immunodominant portion of their immunoreactive surface antigen: the "immune decoy protein."

One object of one embodiment of this invention includes the location and identification of DNA sequences that code for parasitic antigen-like peptides or polypeptides useful in the vaccines of this invention, the transformation of a variety of hosts with these DNA sequences and the production in those transformed hosts of parasitic antigen-like peptides or polypeptides which lack substantially all of the series of repeated amino acids which characterize the naturally occurring epitope. Among the parasitic antigen-like peptides, polypeptides and immunogens produced according to this invention are modified protozoan antigens, such as modified plasmodial or modified Babesia antigens.

Expression of such DNA sequences in appropriate hosts permits the production in large quantities of peptides and polypeptides which are useful in the vaccines of this invention. The molecular structure and properties of these peptides and polypeptides may thus be readily determined and their effectiveness in binding to and blocking the activity of the target parasite may be readily assayed. The peptides and polypeptides are useful, either as produced in the host or after appropriate derivatization or modification, in compositions and methods for detecting and improving the production of these products for use in the vaccination of target hosts, including humans.

The repeat-deleted parasitic surface antigen peptides and polypeptides of the present invention, may also be prepared using methods for making synthetic peptides which are known to the art. In addition, other known methods of making immunogens, including anti-idiotypic antibody formation, may be employed.

## BRIEF DESCRIPTION OF THE DRAWINGS

Figure 1 depicts the nucleotide sequence and derived amino acid sequence of the malarial insert of p31-1.

Figure 2 is a schematic depiction of the construction of the recombinant DNA molecule 31-1 longer Delete.

Figure 3 is a schematic depiction of the construction of the recombinant DNA molecule 31-1 Repeat Deleted.

Figure 4 depicts the nucleotide sequence and derived amino acid sequence of 31-1 Repeat Deleted.

Figure 5 depicts the nucleotide sequence of expression vectors 31a, 31b, 31c and 34c.

## BEST MODE OF CARRYING OUT THE INVENTION

In order that the invention herein described may be more fully understood, the following detailed description is set forth.

In this description the following term is employed:

REPEATED SEQUENCE --As used in this application, the term "repeated sequence" or "repitope" refers to a tandemly repeating epitope of a parasitic surface antigen. This includes, a series of two or more amino acids, within a polypeptide, which is tandemly repeated at least twice within each molecule. For example, the 19 tandem repeats of nine amino acids in the circumsporozoite protein of P. vivax, and the 12 amino acids repeated 12 times in P. Knowlesi, exemplify the repeated sequences, or repitopes referred to in this description.

The present invention relates to vaccines against protozoan parasites which are believed to avoid detection by the immune system through the presence of a repeated amino acid epitope or repitope.

According to the present invention peptides or polypeptides derived from the surface antigen of the target parasite, from which all or part of the repitope has been removed, are used to immunize against or treat infection by protozoan parasites such as malaria and Babesia.

In one embodiment of the present invention, DNA sequences that code for protozoan parasitic antigens are identified and that portion of the DNA sequence which codes for the repeated amino acids of the antigen is either substantially or completely deleted. These DNA sequences can be employed in one embodiment in this invention to produce those "repeat-deleted" parasitic antigens on expression in various prokaryotic and eukaryotic hosts transformed with them for use in the vaccines of the present invention. These DNA sequences may also be modified so as to produce peptides or polypeptides on either or both sides of the repitope.

Alternative methods of making these peptides or polypeptides are known to those of skill in the art. They include, for example, synthetic peptide production of the peptides or polypeptides on either or both sides of the repitope. In addition, various immunologic methods of making vaccines can be used to form the peptides or polypeptides of the present invention.

Administration of these compositions, or pharmaceutically acceptable and immunologically effective derivatives thereof, may be via any of the conventionally accepted modes of administration of agents which exhibit immunogenicity against protozoan parasites. These include oral or parenteral administration.

The compositions used in these vaccines may also be in a variety of forms. These include, for example solid, semi-solid and liquid dosage forms, such as powder, liquid solutions or suspensions, suppositories, injectable and infusable solutions. The preferred form depends on the intended mode of administration and therapeutic application.

The compositions also will preferably include conventional pharmaceutically acceptable carriers and may include other medicinal agents, carriers, adjuvants, excipients, etc., e.g., human serum albumin or plasma preparations. Preferably, the compositions of the invention are in the form of a unit dose. The amount of active compound administered as a vaccination or as a medicament at one time, or over a period of time, will depend on the subject being treated, the manner and form of administration, and the judgement of the treating physician. However, an effective dose may be in the range of from about 1 ng to about 1 mg of the composition of this invention, preferably about 100 μg to about 500 μg; it being recognised that lower and higher doses may also be useful.

Accordingly, this invention provides a method of vaccination against and a method of treatment of protozoan parasitic infection in mammals, including humans, comprising the adminstration of an immunologically effective amount of the vaccine or medicament characterized by the peptides or polypeptides of the present invention.

In order that our invention herein described may be more fully understood, the following example is set forth. It should be understood that this example is for illustrative purposes only and should not be construed as limiting this invention in any way to the specific embodiment recited therein.

Example

In this example we demonstrate one method of making a polypeptide containing the unique sequence of the surface antigen of P.falciparum, but lacking the immunodominant repeat sequences which may allow the parasite to evade and decoy the immune system.

We first produced a stage-specific late - schizont-merozoite antigen 31-1 of P. falciparum as described in PCT application WO85/03725 using the DNA insert of p31-1, and then modified that DNA sequence to delete the entire sequence coding for the repitope. As pointed out above, the repitope may alternatively be partially deleted or polypeptides on either side of it prepared and still result in compositions useful as effective immunogens.

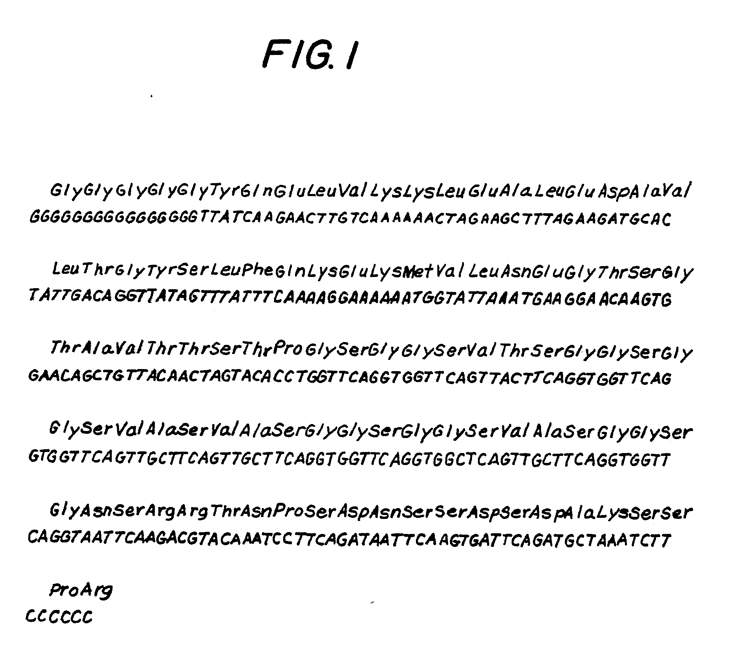

The nucleotide sequence of the DNA insert of p31-1 and the amino acid sequence derived therefrom are depicted in Figure 1. As depicted in Figure 1, the amino acid sequence derived from the DNA insert p31-1 is divided into three areas: 43 amino acids of unique sequence, 30 amino acids of tripeptide repeats (ser-gly-gly or ser-val-ala) and a downstream unique sequence of 20 amino acids. A recombinant DNA molecule containing the malarial insert of p31-1 has been deposited in the Deutsche Sammlung von Microorganismen, Gottingen, West Germany, and identified there as PF-13: E.coli 537 (pPL31A-Pst (Pst)/X11-011) [DSM 2890] and PF-14: E.coli K12λ (pPL31A-Pst(Pst)/1) [DSM 2891].

We had previously observed that the repeats "Ser-Gly-Gly" of p31-1 (see PCT application WO85/03725) are more immunogenic than the unique regions. However, we also observed that the same repeat sequence does not exist in every isolate of p31-1, while the adjacent sequences remain constant. As discussed above, it has been postulated that the parasite avoids attack by the host's immune system when the vaccines used to elicit an immune response against the parasite comprise polypeptides which include the variable repeat sequences. Therefore, we decided to prepare a vaccine comprising polypeptides which lack these repeats.

To this end, we first made a series of constructions that very precisely deleted the repeated sequence of our original "31-1" sequence (Figure 1). For these constructions, we first used p31-1 as a hybridization probe to screen a genomic malarial library, prepared in cloning vector pUC9 and E.coli JM103 and selected the following related DNA sequences:

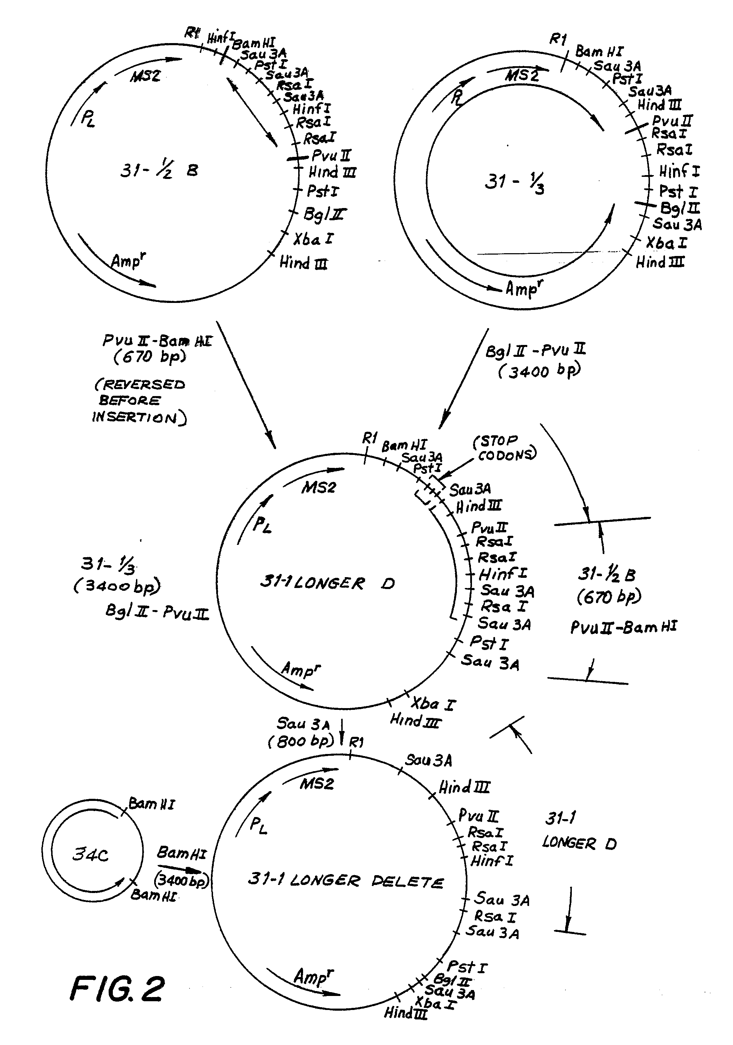

(a) plasmid 31-1/2 B in which the cDNA has been synthesized backwards in comparision with 31-1, and 31-1/3 so that the gene is oriented on the anti-sense direction. This plasmid has an 18G tail after the first Pstl site and a 17C tail before the second Pstl site (Figure 2).

(b) plasmid 31-1/3 which has a 23G tail downstream of the first Pstl site and a 17C tail upstream of the second Pstl site (Figure 2).

As depicted in Figure 2, we then subjected p31-1/3 to Bglll and Pvull digestion and isolated the longer (3400 bp) fragment. And, we subjected 31-1/2B to digestion by Pvull and BamHl and isolated the shorter (670 bp) fragment.

Referring now to Figure 2, we constructed plasmid "31-1 longer D" by ligating the shorter Pvull-BamHl fragment (670 bp) of the 31-1/2 B plasmid to the longer Bglll-Pvull fragment (3400 bp) of the 31-1/3 plasmid. The fragment taken from 31-1/2B was reversed prior to ligating. The resulting construction includes the entire 31-1 gene; however, no expression was possible with this construction because of the stop codons occurring between the MS2 sequence of the vector and the start codon of the 31-1 gene (95 bp in front of the Hindlll site). As can be seen from Figure 2, both the Baml and the Bglll sites were deleted after ligation.

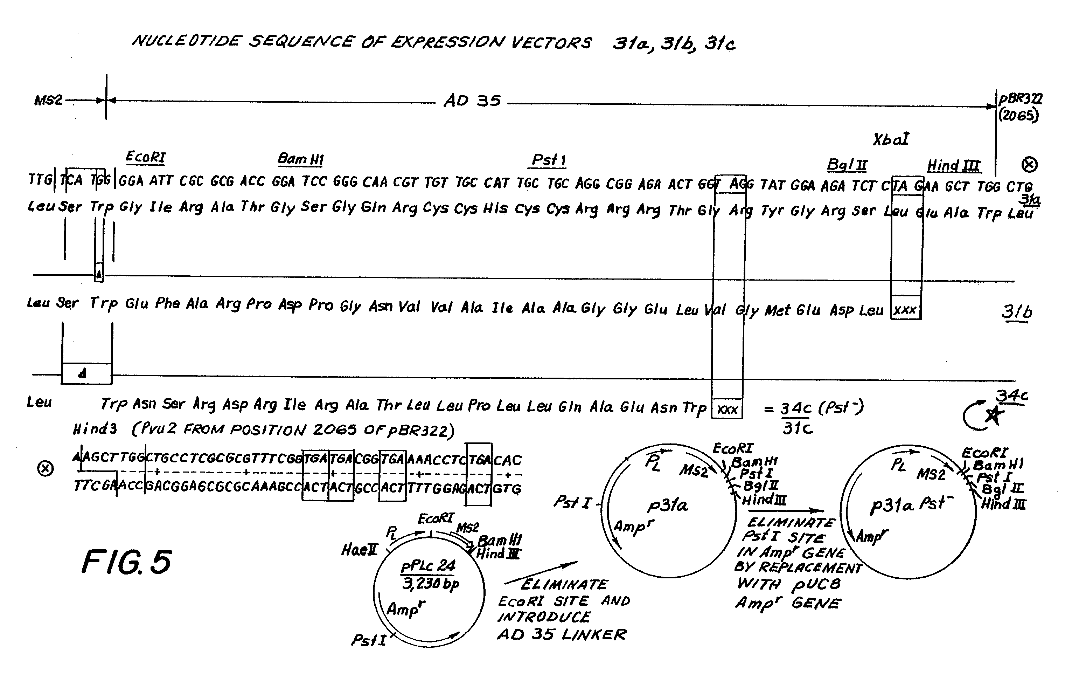

As also depicted in Figure 2, we digested this "31-1 longer D" plasmid with Sau 3A and isolated the shorter (800 bp) fragment. By this digestion, we removed all the left part of 31-1 longer D containing the stop codon, up to the first Sau 3A site, as well as the right part starting at the third Sau 3A site. We used the 34C plasmid as a vector after digesting it with BamHl. Expression vector 34C is a derivative of pPLc24 [E. Remaut et al., Gene, 15, pp. 81-93 (1981)] and is related to expression vector p31a, which is described in WO85/03725 as pPL31A-Pst. Like pPL31A-Pst, plasmid 34C derives from pPLc24 in which an AD35 linker is introduced. However 34C does not have a Pst I restriction endonuclease recognition site downstream of the coding sequence for the MS2 portion of the vector. Expression vector 34C differs from pPL31A-Pst in a deletion of four base pairs at the end of the MS2 coding region and a deletion of 27 base pairs from the end of the AD35 linker (see Figure 5).

We then constructed plasmid "31-1 longer Delete" by ligating this shorter fragment (~800 bp) of clone 31-1 longer D to the longer fragment (~3400 bp) of plasmid 34C which was isolated after Bam HI digestion. As can be seen from Figure 2, this insertion resulted in the elimination of both BamHl sites.

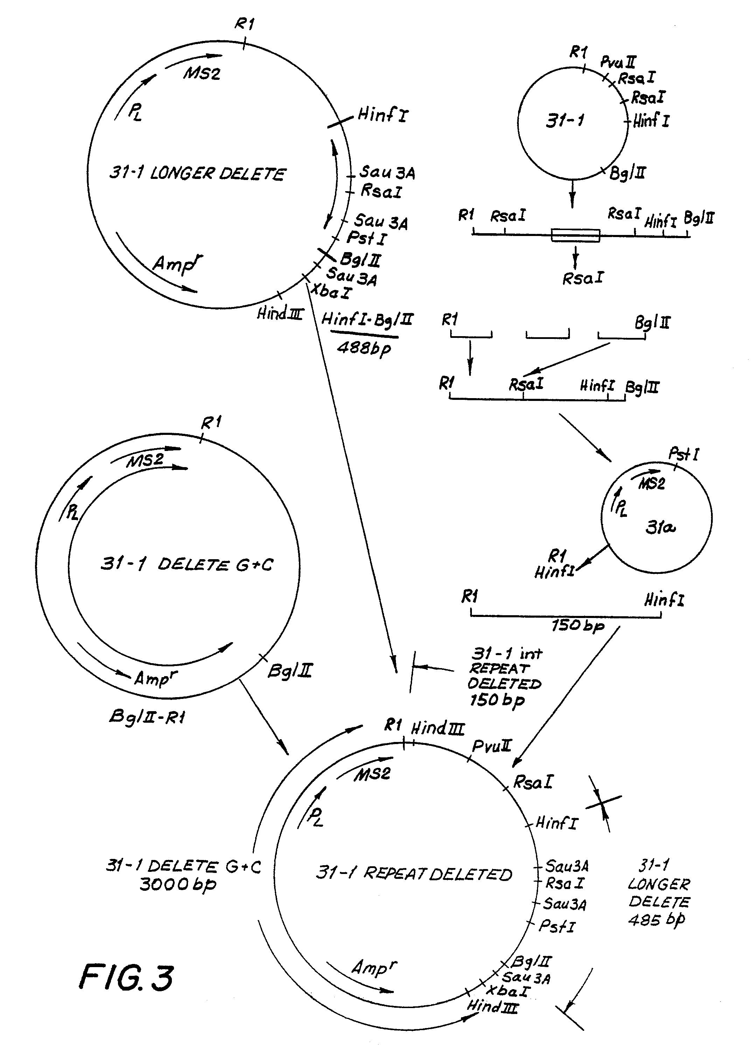

As depicted in Figure 3, we then created plasmid "31-1 Repeat Deleted" by ligating the following three fragments: (1) the 150 bp EcoRI-Hin fl fragment of 31.1 int Repeat Deleted, (2) the 488 bp Hin fl-Bglll fragment of 31.1 longer Delete, and (3)

the 3000 bp Bglll-EcoRI fragment of 31.1 Delete G + C. This construction (31-1 Repeat Deleted) resembles the plasmid 31.1 longer Delete, but it lacks the repeated sequence.

In order to construct 31-1 Repeat Deleted, we first subjected plasmid 31-1 longer Delete to digestion by Hinfl and Bglll and excised the smaller (488 bp) fragment.

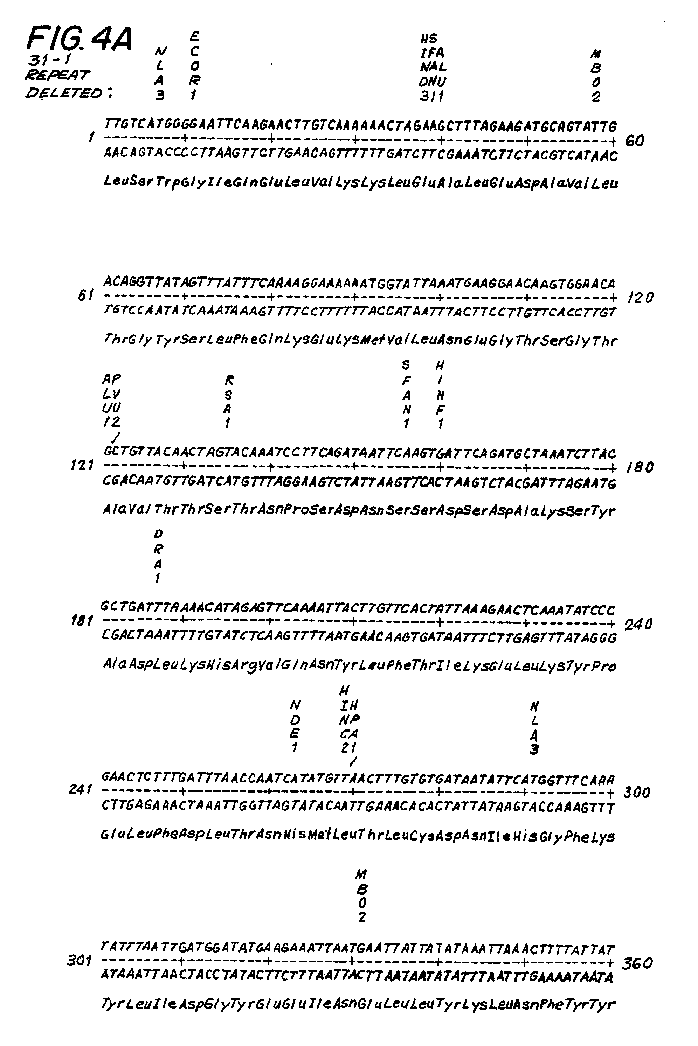

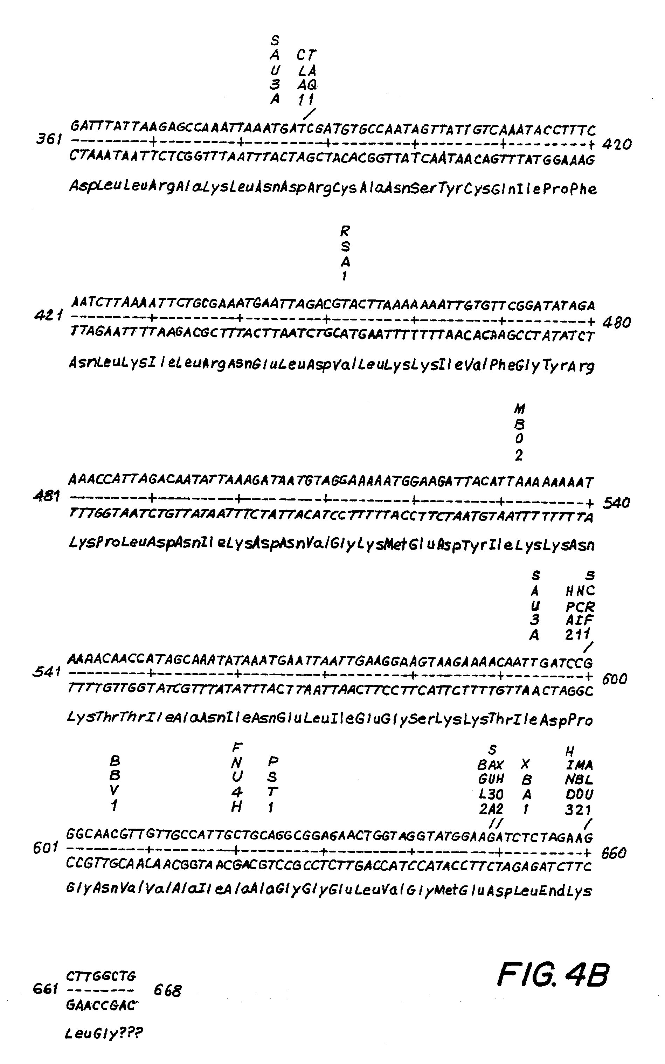

We then deleted 80 bases from the plasmid p31.1 to create "31.1 int Repeat Deleted" thereby deleting the repetitive part of the sequence Ser-Gly-Gly. We deleted the 80 bases by subjecting p31-1 to EcoRI and Bglll digestion and isolating the shorter EcoRI-Bglll fragment. We subjected that fragment to Rsal digestion and excised three fragments of varying length. The first fragment and the third fragment were annealed to create an EcoRI-Bglll fragment which lacks the repitope. By deleting this central nucleotide fragment which contained the coding sequence for the 10 repeated amino acid sequence of the antigen, we created a DNA sequence coding for a shorter protein with no repeats (see Figure 4).

We inserted this EcoRI-Bglll fragment into p31a. Expression vector p31a, as described above, is related to p34c, which are both derivatives of pPLc24. P31a is characterized by a Pstl restriction site downstream of the coding sequence for the MS2 portion of the vector, in which to insert a cDNA sequence for expression under control of the P<sub>L</sub>promoter of the vector (Figure 5). We then subjected the plasmid to digestion by EcoRI and Hin fl in order to delete the terminal nucleotides.

We then obtained the last fragment needed to create 31-1 Repeat Deleted from "31.1 Delete G + C" a derivative of clone 31-1, by first deleting the G and C tails of the DNA sequence depicted in Figure 1 (p31-1). Using site-specific mutagenesis, we then created an EcoRI site at nucleotides 12-15, i.e., in front of the repeats, and a Bglll site behind the repeats, at nucleotides 282-287. The oligonucleotides linkers used were:
MALBglll GATTCAGATCTTAAATCTT
MALEcoRI GGGGGGAATTCAAGAACTT

Finally, we annealed these three fragments (31.1 longer Delete, 31.1 int Repeat Deleted, and 31.1 Delete G + C) to construct plasmid 31-1 Repeat Deleted. We ligated the "repeat-deleted" EcoRI-Hinfl fragment (150 bp) with the Hinfl end piece of the 488 bp fragment of 31.1 longer Delete. We then inserted these fragments into the 3000 bp of 31.1 Delete G + C.

## EXPRESSION IN E.COLI

We introduced the expression plasmid 31-1 Repeat Deleted into E.coliK12 to assay the production of modified malarial antigen polypeptide.

We analyzed total cell aliquots of each fermentation mixture for protozoan parasitic protein production by SDS-polyacrylamide gel electrophoresis. We performed a densitometer scan of a portion of the stained gel which represented the malaria protein production of an aliquot taken after harvesting. This scan indicated that 10-15% of the total protein was the antigen.

## USE OF THE ANTIGENS OF THIS INVENTION IN METHODS AND COMPOSITIONS FOR PROTECTING AGAINST AND TREATING PROTOZOAN PARASITIC INFECTIONS

The immunogens of this invention may be employed in a variety of pharmaceutically acceptable compositions to protect against and treat protozoan parasitic infections. Such compositions may include various pharmaceutically acceptable carriers or adjuvants. They may also include compounds that enhance the immune response. These may include, for example various lymphokines and interferons. These mixtures may be the most effective in protecting humans against and treating malarial and other protozoan parasitic infections.

These compositions may be used in a variety of pharmaceutically acceptable dosage forms and means of treatment well known in the vaccine art.

Microorganisms and DNA sequences for use in the production of polypeptides in accordance with one embodiment of this invention are exemplified by a culture deposited in the Deutsche Sammlung von Microorganismen, Gottingen, West Germany, on December 27, 1985 and identified there as:
E.coli K12 (SG936cl) (p31-1 Repeat Delete)
It has been assigned accession number DSM 3618.

While we have hereinbefore presented a number of embodiments of our invention, it is apparent that our basic construction may be altered to provide other embodiments which utilize the processes and compositions of this invention. Therefore, it will be appreciated that the scope of this invention is to be defined by the claims appended hereto, rather than the specific embodiments which have been presented hereinbefore by way of example.

## Claims

1. A composition for use as a vaccine against or a medicament for treating protozoan parasitic infection wherein the parasite is characterized by a surface antigen having a repitope, which composition comprises an immunologically effective amount of a peptide or polypeptide characterized by a substantial or complete deletion of the repitope which characterizes said antigen.

2. The composition according to claim 1, wherein the protozoan parasitic infection is malaria.

3. A peptide or polypeptide comprising a sequence of amino acids having sufficient immunogenicity to elicit an immune response to a target protozoan parasite, said parasite being characterized by a surface antigen having a repitope, said sequence being selected from the group consisting of the sequence of the surface antigen of said parasite adjacent to one side of the repitope of that antigen, the sequence of the surface antigen of said parasite adjacent to the other side of the repitope of that antigen, fragments of either of those sequences and combinations of any of the adjacent sequences and fragments.

4. A peptide or polypeptide according to claim 3, wherein the sequence comprises the sequence adjacent to both sides of the repitope which characterizes the surface antigen of the target protozoan parasite.

5. A process for producing the peptide or polypeptide according to anyone of claims 3 or 4 comprising the step of culturing a microbial host transformed with a recombinant DNA molecule comprising a DNA sequence encoding a protozoan parasitic antigenlike peptide or polypeptide, said DNA sequence being characterized by a substantial or complete deletion of the coding sequence for the repitope which characterizes the targeted parasitic antigen.

6. The process according to claim 5, wherein the parasitic antigen is a malarial antigen.

7. The process according to claim 6, wherein the host is selected from the group consisting of E.coli K12, E.coli SG936, E.coli SG935, E.coli SG928, E.coli SG927, and other E.coli strains which are lon mutants and which are characterized by their production of low levels of lon protease.

8. A method for immunizing against or treating infections characterized by protozoan parasites, said parasites being characterized by a surface antigen containing a repitope, said method comprising administering an immunologically effective amount of a composition according to claim 1.

Claims for the following contracting states: Austria, Spain and Greece:

1. A process for producing a peptide or polypeptide comprising a sequence of amino acids having sufficient immunogenicity to elicit an immune response to a target protozoan parasite, said parasite being characterized by a surface antigen having a repitope, said sequence being selected from the group consisting of the sequence of the surface antigen of said parasite adjacent to one side of the repitope of that antigen, the sequence of the surface antigen of said parasite adjacent to the other side of the repitope of that antigen, fragments of either of those sequences and combinations of any of the adjacent sequences and fragments, said process comprising the step of culturing a microbial host transformed with a recombinant DNA molecule comprising a DNA sequence encoding a protozoan parasitic antigenlike peptide or polypeptide, said DNA sequence being characterized by a sequence for the repitope which characterizes the targeted parasitic antigen.

2. The process according to claim 1, wherein the sequence comprises the sequence adjacent to both sides of the repitope which characterizes the surface antigen of the target protozoan parasite.

3. The process according to claim 1, wherein the parasitic antigen is a malarial antigen.

4. The process according to claim 1, wherein the host is selected from the group consisting of E.coli K12, E.coli SG936, E.coli SG935, E.coli SG928, E.coli SG927, and other E.coli strains which are lon mutants and which are characterized by their production of low levels of lon protease.

# FIG. 1

GlyGlyGlyGlyGlyTyrGlnGluLeuValLysLysLeuGluAlaLeuGluAspAlaVal
GGGGGGGGGGGGGGGGGTTATCAAGAACTTGTCAAAAAACTAGAAGCTTTAGAAGATGCAC

LeuThrGlyTyrSerLeuPheGlnLysGluLysMetValLeuAsnGluGlyThrSerGly
TATTGACAGGTTATAGTTTATTTCAAAAGGAAAAAATGGTATTAAATGAAGGAACAAGTG

ThrAlaValThrThrSerThrProGlySerGlyGlySerValThrSerGlyGlySerGly
GAACAGCTGTTACAACTAGTACACCTGGTTCAGGTGGTTCAGTTACTTCAGGTGGTTCAG

GlySerValAlaSerValAlaSerGlyGlySerGlyGlySerValAlaSerGlyGlySer
GTGGTTCAGTTGCTTCAGTTGCTTCAGGTGGTTCAGGTGGCTCAGTTGCTTCAGGTGGTT

GlyAsnSerArgArgThrAsnProSerAspAsnSerSerAspSerAspAlaLysSerSer
CAGGTAATTCAAGACGTACAAATCCTTCAGATAATTCAAGTGATTCAGATGCTAAATCTT

ProArg
CCCCCC

0 254 862

FIG. 2

FIG.3

## FIG.4A

```
31-1        N     E                              HS                        M
REPEAT      L     C                              IFA                       B
DELETED:    A     O                              NAL                       O
            3     R                              DNU                       2
                  1                              311
```

```
    TTGTCATGGGGAATTCAAGAACTTGTCAAAAAACTAGAAGCTTTAGAAGATGCAGTATTG
1   ---------+---------+---------+---------+---------+---------+  60
    AACAGTACCCCTTAAGTTCTTGAACAGTTTTTTGATCTTCGAAATCTTCTACGTCATAAC

    LeuSerTrpGlyIleGlnGluLeuValLysLysLeuGluAlaLeuGluAspAlaValLeu
```

```
    ACAGGTTATAGTTTATTTCAAAAGGAAAAAATGGTATTAAATGAAGGAACAAGTGGAACA
61  ---------+---------+---------+---------+---------+---------+  120
    TGTCCAATATCAAATAAAGTTTTCCTTTTTTACCATAATTTACTTCCTTGTTCACCTTGT

    ThrGlyTyrSerLeuPheGlnLysGluLysMetValLeuAsnGluGlyThrSerGlyThr
```

```
    AP              R               S      H
    LV              S               F      I
    UU              A               A      N
    12              1               N      F
    /                               1      1
    GCTGTTACAACTAGTACAAATCCTTCAGATAATTCAAGTGATTCAGATGCTAAATCTTAC
121 ---------+---------+---------+---------+---------+---------+  180
    CGACAATGTTGATCATGTTTAGGAAGTCTATTAAGTTCACTAAGTCTACGATTTAGAATG

    AlaValThrThrSerThrAsnProSerAspAsnSerSerAspSerAspAlaLysSerTyr
```

```
            D
            R
            A
            1
    GCTGATTTAAAACATAGAGTTCAAAATTACTTGTTCACTATTAAAGAACTCAAATATCCC
181 ---------+---------+---------+---------+---------+---------+  240
    CGACTAAATTTTGTATCTCAAGTTTTAATGAACAAGTGATAATTTCTTGAGTTTATAGGG

    AlaAspLeuLysHisArgValGlnAsnTyrLeuPheThrIleLysGluLeuLysTyrPro
```

```
                        H
            N           IH                          N
            D           NP                          L
            E           CA                          A
            1           21                          3
                        /
    GAACTCTTTGATTTAACCAATCATATGTTAACTTTGTGTGATAATATTCATGGTTTCAAA
241 ---------+---------+---------+---------+---------+---------+  300
    CTTGAGAAACTAAATTGGTTAGTATACAATTGAAACACACTATTATAAGTACCAAAGTTT

    GluLeuPheAspLeuThrAsnHisMetLeuThrLeuCysAspAsnIleHisGlyPheLys
```

```
                        M
                        B
                        O
                        2
    TATTTAATTGATGGATATGAAGAAATTAATGAATTATTATATAAATTAAACTTTTATTAT
301 ---------+---------+---------+---------+---------+---------+  360
    ATAAATTAACTACCTATACTTCTTTAATTACTTAATAATATATTTAATTTGAAAATAATA

    TyrLeuIleAspGlyTyrGluGluIleAsnGluLeuLeuTyrLysLeuAsnPheTyrTyr
```

```
                              S
                              A     CT
                              U     LA
                              3     AQ.
                              A     11
                                     /
      GATTTATTAAGAGCCAAATTAAATGATCGATGTGCCAATAGTTATTGTCAAATACCTTTC
361   ---------+---------+---------+---------+---------+---------+ 420
      CTAAATAATTCTCGGTTTAATTTACTAGCTACACGGTTATCAATAACAGTTTATGGAAAG

      AspLeuLeuArgAlaLysLeuAsnAspArgCysAlaAsnSerTyrCysGlnIleProPhe
```

```
                              R
                              S
                              A
                              1

      AATCTTAAAATTCTGCGAAATGAATTAGACGTACTTAAAAAAATTGTGTTCGGATATAGA
421   ---------+---------+---------+---------+---------+---------+ 480
      TTAGAATTTTAAGACGCTTTACTTAATCTGCATGAATTTTTTTAACACAAGCCTATATCT

      AsnLeuLysIleLeuArgAsnGluLeuAspValLeuLysLysIleValPheGlyTyrArg
```

```
                                              M
                                              B
                                              0
                                              2

      AAACCATTAGACAATATTAAAGATAATGTAGGAAAAATGGAAGATTACATTAAAAAAAAT
481   ---------+---------+---------+---------+---------+---------+ 540
      TTTGGTAATCTGTTATAATTTCTATTACATCCTTTTTACCTTCTAATGTAATTTTTTTTA

      LysProLeuAspAsnIleLysAspAsnValGlyLysMetGluAspTyrIleLysLysAsn
```

```
                                              S           S
                                              A           HNC
                                              U           PCR
                                              3           AIF
                                              A           211
                                                            /
      AAAACAACCATAGCAAATATAAATGAATTAATTGAAGGAAGTAAGAAAACAATTGATCCG
541   ---------+---------+---------+---------+---------+---------+ 600
      TTTTGTTGGTATCGTTTATATTTACTTAATTAACTTCCTTCATTCTTTTGTTAACTAGGC

      LysThrThrIleAlaAsnIleAsnGluLeuIleGluGlySerLysLysThrIleAspPro
```

```
                     F                              S       H
      B              N       P                      BAX  X  IMA
      B              U       S                      GUH  B  NBL
      V              4       T                      L30  A  DOU
      1              H       1                      2A2  1  321
                                                     //       /
      GGCAACGTTGTTGCCATTGCTGCAGGCGGAGAACTGGTAGGTATGGAAGATCTCTAGAAG
601   ---------+---------+---------+---------+---------+---------+ 660
      CCGTTGCAACAACGGTAACGACGTCCGCCTCTTGACCATCCATACCTTCTAGAGATCTTC

      GlyAsnValValAlaIleAlaAlaGlyGlyGluLeuValGlyMetGluAspLeuEndLys
```

**FIG. 4B**

```
      CTTGGCTG
661   --------  668
      GAACCGAC

      LeuGly???
```

NUCLEOTIDE SEQUENCE OF EXPRESSION VECTORS  31a, 31b, 31c

MS2 ———|————————————— AD 35 —————————————————————————|pBR322
(2065)

FIG.5

European Patent
Office

**PARTIAL EUROPEAN SEARCH REPORT**
which under Rule 45 of the European Patent Convention
shall be considered. for the purposes of subsequent
proceedings, as the European search report

Application number

EP 87 10 8867.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁴) |
|---|---|---|---|
| X | EP - A - 0 166 410 (UNITED STATES OF AMERICA) <br> * page 6, lines 1-14; page 10, lines 14-34; page 17, lines 1-8 * <br><br> -- <br><br> EP - A - 0 192 626 (SMITHKLINE BECKMAN CORP.) | 1-4 | A 61 K 39/015 <br><br> C 12 N 15/00 <br><br> C 12 P 21/00 |
| X,P | * page 3, lines 1-36; page 4, lines 1-23 * | 1-4 | |
| A,P | * page 6, lines 1-36; page 7, lines 1-13* | 5-7 | |
| | -- | | |
| X,P | EP - A - 0 191 748 (SMITHKLINE BECKMAN CORP.) <br><br> * page 3, lines 20-34; page 4, lines 1-23 * <br><br> -- <br><br> ../.. | 1-4 | |

TECHNICAL FIELDS SEARCHED (Int. Cl⁴)

## INCOMPLETE SEARCH

The Search Division considers that the present European patent application does not comply with the provisions of the European Patent Convention to such an extent that it is not possible to carry out a meaningful search into the state of the art on the basis of some of the claims.
Claims searched completely: 1-7
Claims searched incompletely: -
Claims not searched: 8
Reason for the limitation of the search:

Method for treatment of the human or animal body by
therapy (Art. 52 (4) of EPC)

A 61 K 39/00

C 12 N 15/00

C 12 P 21/00

| Place of search | Date of completion of the search | Examiner |
|---|---|---|
| Berlin | 16-10-1987 | AVEDIKIAN |

EPO Form 1505. 1 03 82

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO 86 01721 (NEW YORK UNIVERSITY)<br>* page 3, lines 13-29, page 4, lines 1-23; claim 1 *<br>-- | 1-4 | |
| A | SCIENTIFIC AMERICAN, vol. 252, no. 5, May 1985, New York, US; G.N. GODSON "Molecular Approaches to Malaria Vaccines"<br>* page 38, column 3, lines 38-50; page 3, column 1, lines 1-3; figure *<br>-- | 1-7 | |
| A,D | NATURE, vol. 305, 1st September 1983, pages 29-33, G.N. GODSON et al.: "Identification and chemical synthesis of a tandemly repeated immunogenic region of Plasmodium knowlesi circumspotorozoite protein"<br>* pages 32-33, conclusion *<br>-- | 1-7 | TECHNICAL FIELDS SEARCHED (Int. Cl.4) |
| A,D | WOA 85 03725 (B. MACH et al.)<br>* claims 17-30 *<br>---- | 1-7 | |